# EUROPÄISCHE PATENTSCHRIFT

(11) **EP 1 525 174 B1**
(45) Veröffentlichungstag und Bekanntmachung des Hinweises auf die Patenterteilung: **23.09.2009**
(21) Anmeldenummer: 03764916.7
(22) Anmeldetag: 05.06.2003
(51) Int. Cl.: C07C 4/06, C07C 11/00, C07C 11/02, C07C 1/06

(54) **VERFAHREN ZUR HERSTELLUNG VON PROPYLEN AUS EINEM C 4 BIS C 8 OLEFINE ENTHALTENDEN EINSATZSTROM**
METHOD FOR PRODUCING PROPYLENE FROM A FLOW CONTAINING C4 TO C 8 OLEFINS
PROCEDE DE PRODUCTION DE PROPYLENE A PARTIR D'UN FLUX CONTENANT DES OLEFINES C4 A C8

(30) Priorität: 19.07.2002 DE 10233069
(43) Veröffentlichungstag der Anmeldung: 27.04.2005
(73) Patentinhaber: Lurgi GmbH, 60439 Frankfurt am Main (DE)
(72) Erfinder: BACH, Hermann, 56412 Heiligenroth (DE); KÖMPEL, Harald, 63263 Neu-Isenburg (DE); AHLERS, Bernd, 61440 Oberursel (DE); TRABOLD, Peter, 64295 Darmstadt (DE); HÖPER, Frank, 45721 Haltern am See (DE)
(74) Vertreter: Meyer-Dulheuer, Karl-Hermann
(86) Internationale Anmeldenummer: PCT/EP2003/005903
(87) Internationale Veröffentlichungsnummer: WO 2004/009519

(56) Entgegenhaltungen:
- EP-A- 0 421 701
- EP-A- 0 511 013
- DE-A- 19 933 063
- US-A- 5 981 819

## Beschreibung

Die Erfindung betrifft ein Verfahren zur Herstellung von Propylen aus einem C₄ bis C₈ Olefine enthaltenden flüssigen Einsatzstrom, der bei 25 bis 200°C verdampft und auf 350 bis 400°C überhitzt wird, der gebildete die Olefine enthaltende Dampf mit heißem Wasserdampf gemischt wird, das Olefine-Dampf-Gemisch bei Eintrittstemperaturen von 450 bis 550°C und Drücken von 0.5 bis 3.0 bar (abs) an einem formselektiven Zeolith-Festbettkatalysator vom Pentasiltyp umgesetzt wird, das dabei gebildete Reaktionsgemisch auf 100 bis 200°C abgekühlt wird und durch eine anschließende weitere Abkühlung auf 40 bis <100°C eine Teilkondensation unter Bildung einer im wesentlichen Ethylen, Propylen, C₄ bis C₈ Olefine und weitere Kohlenwasserstoffe enthaltenden Gasphase und einer im wesentlichen aus Wasser bestehenden in den Einsatzstrom zurückgeführten Flüssigkeitsphase durchgeführt wird.

Um die weltweit steigende Nachfrage nach Propylen, das zu annähernd 98% als Nebenprodukt beim thermischen Kracken (Steamcracking) und beim katalytischen Kracken im Fließbett (Fluid Catalytic Cracking, FCC) von Erdölfraktionen anfällt, befriedigen zu können, ist die Fachwelt bestrebt, Propylene auf sekundäre Weise zu erzeugen. Zu diesem . Zweck werden gemäß Z.:HYDROCARBON ENGINEERING May 1999, S.66/67.C₄₊ Olefine, wie Butylene, Pentene, Hexene etc. mittels eines formselektiven Zeolith-Katalysators vom Pentasil-Typ in "CH₂"-Glieder zerlegt, die sich dann wieder zu Propylen, Ethylen und Butylen in einer Quasi-Gleichgewichtsverteilung rekombinieren, wobei die Umsetzungsrate ca. 83 Gew.-% (42 Gew. % Propylen, 31 Gew. % Butylen und 10 Gew. % Ethylen), bezogen auf Olefine im Einsatzstrom, beträgt. Im Falle einer Rückführung von Butylen in den Prozeßkreislauf ergibt sich sogar eine Ausbeute von 60 Gew. % Propylen und 15 Gew. % Ethylen. Zur Herstellung von Propylen wird der C₄₊Kohlenwasserstoffe enthaltende mit rückgeführtem Butylen gemischte Einsatzstrom nach Durchströmen eines Sättigers, nach Zugabe von Kreislaufwasserdampf in einem Wärmetauscher bei Temperaturen von 20 bis 100° C verdampft, anschließend in einem Wärmetauscher und in einem Ofen auf eine Temperatur von 100 bis 500°C überhitzt und dann einem mit einem formselektiven Zeolith-Festbettkatalysator vom ZSM-5 Typ gefüllten Reaktionsbehälter, vorzugsweise vom Claus-Typ, zugeführt. Der aus dem Reaktionsbehälter austretende Gasstrom wird in einem Wärmetauscher auf eine Temperatur von 100 bis 200°C abgekühlt, auf einen Druck von 2 bis 6 bar (abs) verdichtet, danach der Kondensationsseite eines Verdampfer/Kondensators aufgegeben und die in der Gasphase verbliebenen Kohlenwasserstoffe zu einem Benzin/Olefin-Splitter geleitet, während das vor allem aus Wasser bestehende Kondensat nach einer Druckabsenkung im Verdampfer/Kondensator verdampft und dem Sättiger als Kreislaufwasserdampf zugeführt wird. Die Gasphase wird in dem Splitter in eine C₃-Kohlenwasserstoffe enthaltende Fraktion und Benzin getrennt.

USA 981 819,DE 199 33 063,EP0421701 und EP0511013 offenbaren ein Verfahren zur Herstellung von Propylen aus einem C₄ bis C₇/C₈ Olefine enthaltenden flüssigen Einsatzstrom mittels eines Pentasil.Typ Zeolith-Festbettkatalysators.

Es ist die Aufgabe vorliegender Erfindung, die Ausbeute an Propylen mittels des eingangs beschriebenen Verfahrens ohne Steigerung des technischen Aufwands zu erhöhen.

Gelöst ist diese Aufgabe dadurch, daß die bei der mittels einer Quenchstufe durchgeführten Teilkondensation gebildete Ethylen, Propylen, C₄ bis C₈ Olefine und weitere Kohlenwasserstoffe enthaltende Gasphase auf einen Druck von 20 bis 30 bar (abs) verdichtet wird, die aus der Verdichterstufe austretende Gas- und Flüssigkeitsphase in eine im wesentlichen Propylen, Ethylen und andere leichte Kohlenwasserstoffe enthaltende Gasphase und in eine C₄₊ Olefine enthaltende Flüssigkeitsphase getrennt werden und die Flüssigkeitsphase in eine C₄ bis C₆ Olefine enthaltende Fraktion und eine C₇₊ Olefine enthaltende Fraktion getrennt wird.

Eine vorzugsweise Ausgestaltung des Verfahrens ist darin zu sehen, daß der in der Quenchstufe als Kondensat anfallende Wasserstrom wieder verdampft wird, dann auf eine Temperatur von 600 bis 800°C aufgeheizt und in den dampfförmige Kohlenwasserstoffe enthaltenden Einsatzstrom zurückgeführt wird. Durch diese Maßnahme wird der Einsatzstrom auf die für die Reaktionsstufe erforderliche Eingangstemperatur von 450 bis 550°C erhitzt, so daß auf das Aufheizen des Kohlenwasserstoffe enthaltenden Einsatzstroms mittels eines besonderen Ofens verzichtet werden kann.

Nach einem weiteren erfindungsgemäßen Merkmal wird der überwiegende Teil der erzeugten C₄ bis C₆ Olefine in den dampfförmigen Kohlenwasserstoffe enthaltenden Einsatzstrom zurückgeführt, um die Propylenausbeute weiter zu steigern.

Es ist weiterhin von Vorteil, das in der Verdichterstufe anfallende Wasser zu verdampfen, dann den Dampf auf eine Temperatur von 600 bis 800°C aufzuheizen und dem dampfförmigen Kohlenwasserstoffe enthaltenden Einsatzstrom wieder zuzusetzen.

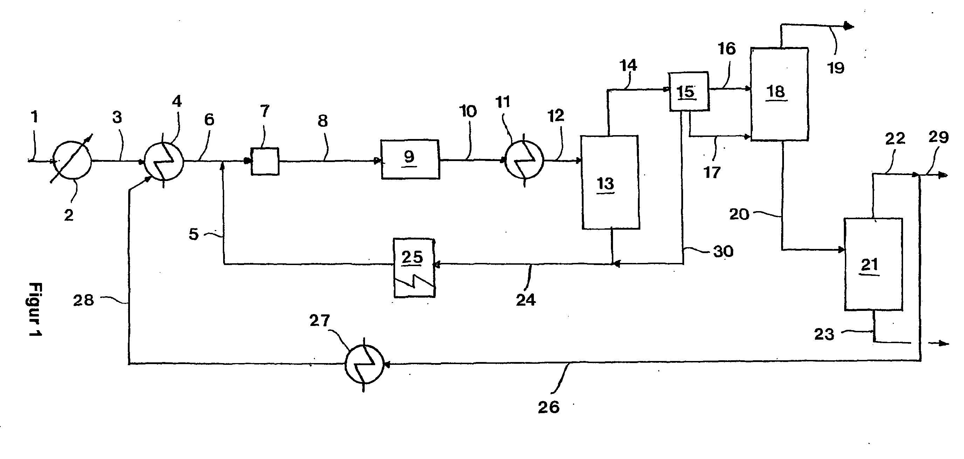

Die Erfindung ist in der Zeichnung durch ein Verfabrensfließbild beispielhaft dargestellt und wird nachstehend näher erläutert.

Über Leitung (1) wird der C₄ bis C₈ Olefine enthaltende Einsatzstrom einem Verdampfer (2) aufgegeben, in diesem auf eine Temperatur von 100°C erwärmt und bei einem Druck von 6.5 bar (abs) verdampft. Der Einsatzstrom strömt über Leitung (3) in einen Überhitzer (4) und wird in diesem auf eine Temperatur von 350°C überhitzt. Zur weiteren Überhitzung des Einsatzstroms auf eine Temperatur von 500°C wird dieser mit über Leitung (5) zugeführtem und in Leitung (6) eingespeisten 700°C heißen Wasserdampf mittels der Mischvorrichtung (7) gemischt Über Leitung (8) wird der eine Temperatur von 500° C besitzende Gasstrom in den Reaktor (9) geleitet. In einer endothermen adiabaten Reaktion an einem Zeolith - Festbettkatalysator wird der überwiegende Teil der C₄ bis C₈ Olefine zu C₃ bis C₆ Olefinen on mit Propylen als Hauptkomponente umgesetzt. Das eine Reaktionstemperatur von 460°C aufweisende über Leitung (10) abgeführte Reaktionsgemisch wird in dem Wärmetauscher (11) auf eine Temperatur von 200°C abgekühlt. Anschließend wird das Reaktionsgemisch über Leitung (12) einer Quenchkolonne (13) aufgegeben, in der eine Abkühlung auf 60° C am Kolonnenkopf erfolgt. Die in der Quenchkolonne (13) anfallende Gasphase wird über Leitung (14) einem Kompressor (15) zugeführt, auf einen Druck von 27 bar (abs) verdichtet und auf eine Temperatur von 60° C abgekühlt. Die im Kompressor (15) gebildeten Kohlenwasserstoffe enthaltenden Gas- und Flüssigkeitsphase werden über Leitung (16) bzw. (17) einer Destillationskolonne (18) zugeleitet, über deren Kopfüber Leitung (19) die eine Temperatur von 20°C aufweisende Propylen, Ethylen und andere leichte Kohlenwasserstoffe enthaltende Gasphase und aus deren Sumpf eine im wesentlichen C₄₊ Olefine enthaltende Flüssigkeitsphase über Leitung (20) abgeführt werden. Das Kopfprodukt besitzt einen Propylengehalt von ca. 75 Gew.-%. Das Sumpfprodukt wird einer Destillationskolonne (21) zugeführt und in dieser in eine C₄ bis C₆ Olefine enthaltende Gasphase mit einer Temperatur von 50°C und eine C₇₊ Olefine enthaltende Flüssigkeitsphase getrennt. Die über Leitung (22) aus der Destillationskolonne (21) austretende Gasphase wird ca. 65% über Leitung (26) einem Überhitzer (27) aufgegeben und danach über Leitung (28) in den Wärmetauscher (4) für die Überhitzung des dampfförmigen Einsatzstroms zurückgeführt, während der Rest der Gasphase über Leitung (29) ausgeschleust wird. Die die Destillationskolonne (21) verlassende Flüssigkeitsphase wird über Leitung (23) aus dem Verfahrensprozeß ausgeleitet. Der aus der Quenchkolonne (13) austretende Wasserstrom wird über Leitung (24) in den Verdampfer (25) geleitet und der darin gebildete nach Überhitzung eine Temperatur von 700°C aufweisende Wasserdampf dem eine Temperatur von 350°C besitzenden dampfförmigen Kohlenwasserstoffe enthaltenden Einsatzstrom über Leitung (5) vor dem Einströmen des Einsatzstroms in die Mischvorrichtung (7) zugesetzt. Das im Kompressor (15) gebildete Wasser wird über Leitung (30) in den aus der Quenchkolonne (13) durch Leitung (24) abgeführten Wasserstrom eingespeist.

## Patentansprüche

1. Verfahren zur Herstellung von Propylen aus einem C₄ bis C₈ Olefine enthaltenden flüssigen Einsatzstrom, der bei 25 bis 200°C verdampft und auf 350 bis 400°C überhitzt wird, der gebildete die Olefine enthaltende Dampf mit heißem Wasserdampf gemischt wird, das Olefine-Dampf-Gemisch bei Eintrittstemperaturen von 450 bis 550° C und Drücken von 0.5 bis 3.0 bar (abs) an einem formselektiven Zeolith-Festbettkatalysator (9) vom Pentasiltyp umgesetzt wird, das dabei gebildete Reaktionsgemisch auf 100 bis 200° C abgekühlt und durch eine anschließende weitere Abkühlung auf Temperaturen von 40 bis < 100°C eine Teilkondensation unter Bildung einer im wesentlichen Ethylen, Propylen, C₄ bis C₈ Olefine und weitere Kohlenwasserstoffe enthaltenden Gasphase und eine im wesentlichen aus Wasser bestehende in den Einsatzstrom zurückgeführte Flüssigkeitsphase durchgeführt wird **dadurch gekennzeichnet, dass** die bei der mittels einer Quenchstufe (13) durchgeführten Teilkondensation gebildete Ethylen, Propylen, C₄ bis C₈ Olefine und weitere Kohlenwasserstoffe enthaltende Gasphase auf einen Druck von 20 bis 30 bar (abs) verdichtet wird, die aus der Verdichterstufe (15) austretende Gas- und Flüssigkeitsphase in eine im wesentlichen Propylen, Ethylen und andere leichte. Kohlenwasserstoffe enthaltende Gasphase und eine C₄+Olefine enthaltende Flüssigkeitsphase getrennt werden und die Flüssigkeitsphase in eine C₄ bis C₆ Olefine enthaltende Fraktion und eine C₇+ Olefine enthaltende Fraktion getrennt wird.

2. Verfahren nach Anspruch 1, **dadurch gekennzeichnet, dass** der in der Quenchstufe (13) als Kondensat anfallende Wasserstrom wieder verdampft, dann auf eine Temperatur von 600 bis 800°C aufgeheizt und in den dampfförmigen Kohlenwasserstoffe enthaltenden Einsatzstrom zurückgeführt wird.

3. Verfahren nach einem der Ansprüche 1 und 2, **dadurch gekennzeichnet, dass** der überwiegende Teil der erzeugten C₄ bis C₆ Olefine in den dampfförmigen Kohlenwasserstoffe enthaltenden Einsatzstrom zurückgeführt wird.

4. Verfahren nach einem der Ansprüche 1 bis 3, **dadurch gekennzeichnet, dass** das in der Verdichterstufe (15) anfallende Wasser verdampft, dann auf eine Temperatur von 600 bis 800°C aufgeheizt und in den dampfförmigen Kohlenwasserstoffe enthaltenden Einsatzstrom zurückgeführt wird.

## Claims

1. Method for the production of propylene from a liquid charge stream containing C₄ to C₈ olefins that evaporates at 25 to 200°C and is superhated to 350 to 400°C, the formed vapour containing the olefins is mixed with hot water vapour, the olefins/vapour mixture is converted at inlet temperatures of 450 to 550°C and pressures of 0.5 to 3.0 bar (abs) on a shape-selective, pentasil-type zeolite fixed-bed catalyst (9), the reaction mixture formed there is cooled to 100 to 200°C, and through a subsequent further cooling to temperatures of 40 to <100°C a partial condensation is carried out with formation of a gaseous phase containing substantially ethylene, propylene, C₄ to C₈ olefins and further hydrocarbons and a liquid phase that is substantially comprised of water and is returned to the charge stream, **characterized in that** the gaseous phase containing ethylene, propylene, C₄ to C₈ olefins and further hydrocarbons that is formed during the partial condensation carried out by means of a quenching step (13) is compressed to a pressure of 20 to 30 bar (abs), the gaseous and liquid phases emerging from the compression step (15) are separated into a gaseous phase containing substantially propylene, ethylene and other light hydrocarbons and a liquid phase containing C₄₊ olefins, and the liquid phase is separated into a fraction containing C₄ to C₆ olefins and a fraction containing C₇₊ olefins.

2. Method according to Claim 1, **characterized in that** the water stream accumulated as condensate in the quenching step (13) is re-evaporated, then heated to a temperature of 600 to 800°C, and returned to the vaporous charge stream containing hydrocarbons.

3. Method according to one of Claims 1 and 2, **characterized in that** the majority of the generated C₄ to C₆ olefins is returned to the vaporous charge stream containing hydrocarbons.

4. Method according to one of Claims 1 to 3, **characterized in that** the water that accumulates in the compression step (15) is evaporated, then heated to a temperature of 600 to 800°C, and returned to the vaporous charge stream containing hydrocarbons.

## Revendications

1. Procédé de préparation de propylène à partir d'un flux de départ liquide contenant des oléfines C₄ à C₈ et étant évaporé à 25 à 200 °C et étant surchauffé à 350 à 400 °C, la vapeur contenant les oléfines ainsi formées étant mélangée avec de la vapeur d'eau chaude, le mélange oléfines-vapeur étant soumis à une réaction sur un catalyseur à lit fixe de zéolithes de type pentasil, les températures d'entrée étant comprises entre 450 et 550°C et les pressions entre 0,5 et 3,0 bar (abs), le mélange réactionnel ainsi formé étant refroidi à 100 à 200 °C pour ensuite effectuer, en le refroidissant à des températures comprises entre 40 et <100 °C, une condensation partielle en obtenant une phase gazeuse contenant essentiellement de l'éthylène, du propylène, des oléfines C₄ à C₈ et d'autres hydrocarbures et une phase liquide contenant essentiellement de l'eau et étant recyclée dans le flux de départ, **caractérisé en ce que** la phase gazeuse contenant de l'éthylène, du propylène, des oléfines C₄ à C₈ et d'autres hydrocarbures et étant obtenue par une condensation partielle effectuée au moyen d'un étage de refroidissement (13) est comprimée jusqu'à l'obtention d'une pression comprise entre 20 et 30 bar (abs), que la phase liquide et gazeuse à l'issue de l'étage de compression (15) est séparée en une phase gazeuse contenant essentiellement du propylène, de l'éthylène et d'autres hydrocarbures légers et en une phase liquide contenant des oléfines C₄₊ et que ladite phase liquide est séparée en une fraction contenant des oléfines C₄ à C₆ et en une fraction contenant des oléfines C₇₊.

2. Procédé selon la revendication 1, **caractérisé en ce que** le flux d'eau obtenu comme condensat dans l'étage de refroidissement (13) est de nouveau évaporé puis chauffé à une température comprise entre 600 et 800 °C et recyclé dans le flux de départ sous forme de vapeur contenant des hydrocarbures.

3. Procédé selon l'une des revendications 1 et 2, **caractérisé en ce qu'**une proportion majoritaire des oléfines C₄ à C₆ obtenues est recyclée dans le flux de départ sous forme de vapeur contenant des hydrocarbures.

4. Procédé selon l'une des revendications 1 à 3, **caractérisé en ce que** l'eau obtenue dans l'étage de compression (15) est évaporée puis chauffé à une température comprise entre 600 et 800 °C et recyclée dans le flux de départ sous forme de vapeur contenant des hydrocarbures.
